# DEMANDE DE BREVET EUROPEEN

(11) **EP 1 201 220 A1**
(43) Date de publication de la demande: **02.05.2002**
(21) Numéro de dépôt: 01402674.4
(22) Date de dépôt: 17.10.2001
(51) Int. Cl.: A61K 7/00, A61K 7/48, A61K 9/50

(54) **Compositions cosmétiques ou pharmaceutiques contenant des microcapsules thermostabilisatrices**

(30) Priorité: 27.10.2000 FR 0013792; 27.10.2000 FR 0013793
(71) Demandeur: L'OREAL, 75008 Paris (FR)
(72) Inventeur: Biatry, Bruno, 94300 Vincennes (FR); Mondet, Jean, 93600 Aulnay Sous Bois (FR); Bara, Isabelle, 75013 Paris (FR); Afriat, Isabelle, 75003 Paris (FR)
(74) Mandataire: Casalonga, Axel

(57) **Abrégé**

La présente invention concerne l'utilisation de microcapsules thermostabilisatrices renfermant au moins un composé cristallin présentant une enthalpie de fusion (ΔH_{f}), mesurée par analyse enthalpique différentielle, comprise entre 75 et 330 kJ/kg, pour protéger des compositions cosmétiques ou pharmaceutiques contre les effets de variations thermiques, ainsi que les compositions cosmétiques ou pharmaceutiques contenant de telles microcapsules thermostabilisatrices.

## Description

La présente invention concerne l'utilisation de microcapsules thermostabilisatrices renfermant des composés cristallins thermoabsorbants pour protéger des compositions cosmétiques ou pharmaceutiques contre les effets de variations thermiques, ainsi que les compositions cosmétiques ou pharmaceutiques contenant de telles microcapsules thermostabilisatrices.

Dans le domaine de la formulation des compositions cosmétiques et pharmaceutiques, on rencontre fréquemment le problème de l'instabilité rhéologique des compositions cosmétiques lorsque celles-ci sont exposées à des variations de température importantes.

En effet, de nombreuses compositions sont épaissies ou gélifiées de manière thermoréversible, c'est-à-dire une augmentation de la température extérieure tend à les fluidifier ou ramollir et un refroidissement peut se traduire par un durcissement ou épaississement indésirable de la composition.

Ainsi, les propriétés d'application de rouges à lèvres, de crayons de couleur ou de sticks déodorants peuvent se trouver altérées par le fait que leur consistance est devenue trop molle ou trop dure suite à un changement de température. Une crème solaire ayant une viscosité satisfaisante à température ambiante peut se liquéfier excessivement sous l'effet de la chaleur ce qui rend plus difficile son application.

Par ailleurs, les variations rhéologiques peuvent dans certains cas entraîner des défauts irréversibles tels qu'une déformation à chaud d'une composition solide ou une séparation de phases dans le cas de compositions sous forme dispersée. Les émulsions et suspensions peuvent également être déstabilisées lors d'un abaissement de température.

Un cas particulier de compositions cosmétiques ou pharmaceutiques à stabiliser contre les variations thermiques sont les compositions d'imprégnation de lingettes, comme par exemple les lingettes de démaquillage. Ces compositions sont le plus souvent des émulsions huile dans eau. Pour que le procédé d'imprégnation des lingettes par pulvérisation ou par trempage puisse être réalisé dans de bonnes conditions, le lait démaquillant doit être suffisamment fluide. La nécessité d'une faible viscosité interdit l'introduction de quantités importantes de polymères stabilisateurs d'émulsions tels que les produits Carbomer® . Les compositions d'imprégnation ainsi que les lingettes imprégnées sont ainsi particulièrement sensibles aux variations thermiques et en particulier le phénomène de crémage des émulsions (décantation des globules huileux conduisant à la formation d'une phase aqueuse exempte de globules huileux) est favorisé à température élevée.

Il est donc important de trouver une solution pour conserver une bonne stabilité de l'émulsion aux températures élevées tout en restant dans des gammes de viscosité faible permettant l'imprégnation des lingettes selon des techniques usuelles.

Par ailleurs, on rencontre dans le domaine cosmétique de nombreuses situations où il est intéressant de maîtriser les échanges thermiques entre le corps, la composition cosmétique et l'environnement.

Ainsi, l'exposition de la peau humaine à des températures très élevées ou très basses peut entraîner des effets indésirables tels que dessèchement ou rougeurs.

La protection de la peau et des lèvres contre le froid se fait généralement par un apport de matières grasses généralement peu satisfaisant d'un point de vue cosmétique.

La protection de la peau contre des excès de chaleur s'avère également difficile. Une approche connue consiste à ajouter aux compositions du menthol ou des dérivés de celui-ci qui, par un effet biologique, provoquent localement et transitoirement une sensation de fraîcheur. Le contact direct entre le produit chimique (menthol) et la peau peut cependant conduire à des irritations cutanées.

La qualité et la longévité du maquillage dépendent souvent de conditions thermiques particulières et peuvent se trouver fortement altérées dans des conditions de température extrêmes. Les dépôts de produits peuvent soit se liquéfier et couler sous l'action de la chaleur et de la transpiration, soit perdre leur plasticité et leur souplesse en cas de grand froid.

Aucune solution efficace n'a jusqu'ici été apportée aux problèmes énoncés.

La demanderesse a découvert de manière surprenante qu'il était possible d'améliorer sensiblement la stabilité thermique de compositions cosmétiques et pharmaceutiques et de résoudre, au moins partiellement, les problèmes décrits ci-dessus liés aux échanges thermiques dans le domaine cosmétique grâce à l'utilisation de microcapsules particulières, décrites plus en détail ci-dessous, ayant des capacités d'absorption et de restitution de chaleur.

Les microcapsules utilisées dans la présente invention contiennent, de préférence dans une enveloppe étanche, des composés partiellement ou complètement cristallins qui, lorsqu'on les porte à une température proche de leur température de fusion, absorbent une importante quantité de chaleur, appelée chaleur latente de fusion. L'absorption de cette chaleur latente de fusion se traduit par une stabilité de la température du composé malgré l'apport d'énergie thermique. Cet effet est semblable à un effet "tampon" et permet de thermostater, pendant un certain temps et dans un intervalle de température proche de la température de fusion du composé, l'environnement direct des microcapsules malgré une variation de la température extérieure.

La capacité d'absorption de chaleur décrite ci-dessus va de paire avec la possibilité, également très intéressante, de restituer l'énergie absorbée sous forme de chaleur latente de cristallisation lorsque la température est abaissée en dessous du point de fusion du composé encapsulé. Les microcapsules contenant le composé à l'état fondu constituent ainsi une réserve d'énergie thermique.

Les microcapsules ayant des capacités d'absorption et de restitution de chaleur réversibles et capables de stabiliser la température de leur environnement proche seront appelées par la suite "microcapsules thermostabilisatrices".

La présente invention a par conséquent pour objet l'utilisation de microcapsules renfermant au moins un composé cristallin présentant une enthalpie de fusion (ΔH_{f}), mesurée par analyse enthalpique différentielle, comprise entre 75 et 330 kJ/kg, pour protéger des compositions cosmétiques ou pharmaceutiques contre les effets de variations thermiques.

L'invention a également pour objet des compositions cosmétiques ou pharmaceutiques comprenant, dans un support physiologiquement acceptable, des microcapsules renfermant au moins un composé cristallin présentant une enthalpie de fusion (ΔH_{f}), mesurée par analyse enthalpique différentielle, comprise entre 75 et 330 kJ/kg.

L'introduction de microcapsules thermostabilisatrices dans des compositions cosmétiques ou pharmaceutiques permet de protéger celles-ci pendant un certain temps contre des coups de chaud ou de froid, c'est-à-dire de préserver leurs caractéristiques rhéologiques et de prévenir les effets irréversibles liés à des changements de température.

L'efficacité des microcapsules thermostabilisatrices utilisées dans la présente invention pour améliorer la stabilité thermique de compositions cosmétiques ou pharmaceutiques dépend directement de la capacité d'absorption thermique des composés cristallins encapsulés et de leur point de fusion.

Le terme "composé cristallin" tel qu'il est utilisé dans la présente demande englobe les composés partiellement ou totalement cristallins. Le taux de cristallinité des composés utilisés n'est pas déterminant dans la mesure où le composé présente l'enthalpie de fusion cristalline requise pour l'utilisation envisagée.

La capacité d'absorption thermique des microcapsules utilisées est directement proportionnelle à l'enthalpie de fusion du composé cristallin encapsulé. Cette enthalpie de fusion est mesurée par analyse enthalpique différentielle (en anglais : *Differential Scanning Calorimetry*).

L'enthalpie de fusion d'un composé est la quantité d'énergie nécessaire pour transformer un échantillon partiellement ou totalement cristallin en un échantillon totalement amorphe. Le thermogramme ΔCp = f(T), dans lequel ΔCp représente la différence de capacité thermique de l'échantillon par rapport à un échantillon de référence ne subissant aucune transition thermique dans le domaine étudié, présente donc un signal endothermique dont l'aire est proportionnelle à l'enthalpie de fusion (ΔH_{f}) de l'échantillon.

Comme indiqué, les microcapsules utilisées dans les compositions cosmétiques de la présente invention contiennent des composés cristallins ayant une enthalpie de fusion comprise entre 75 et 330 kJ/kg. Cette enthalpie de fusion est de préférence comprise entre 100 et 300 kJ/kg, et idéalement entre 150 et 280 kJ/kg.

Outre la capacité thermique des composés cristallins enfermés dans les microcapsules thermostabilisatrices des compositions cosmétiques ou pharmaceutiques, le point de fusion de ces composés est un paramètre essentiel déterminant le mode d'action, le domaine d'application et l'efficacité des compositions cosmétiques.

Pour la protection des compositions cosmétiques ou pharmaceutiques contre des variations thermiques, la fusion du composé cristallin encapsulé doit avoir lieu à des températures auxquelles les compositions cosmétiques sont susceptibles d'être exposées, à savoir de préférence dans un domaine de température compris entre environ 0 °C et 90 °C.

Lorsqu'il s'agit de protéger des compositions solides telles que des sticks, des crayons ou des bâtons contre un durcissement trop important à froid, le composé encapsulé doit avoir une température de fusion inférieure à la température ambiante, par exemple comprise entre 5 °C et 15 °C, alors que la protection des compositions contre un ramollissement ou une liquéfaction à chaud nécessite des composés encapsulés ayant un point de fusion supérieur à la température ambiante, par exemple compris entre 25 et 60 °C.

En ce qui concerne les échanges thermiques entre la peau et les compositions cosmétiques et pharmaceutiques évoqués dans l'introduction, d'autres aspects doivent être pris en considération pour le choix du point de fusion.

Ainsi, dans un mode de réalisation des compositions cosmétiques et pharmaceutiques de la présente invention la température de fusion (T_{f}) du composé cristallin encapsulé est inférieure à la température ambiante, de préférence comprise entre 0 et 15 °C.

Des microcapsules renfermant des composés cristallins à point de fusion relativement bas dans une crème de protection permettent de protéger la peau contre l'agression par le froid grâce à la libération de la chaleur latente de cristallisation du composé encapsulé lorsque la température extérieure descend en-dessous du point de fusion du composé cristallin encapsulé.

Dans un autre mode de réalisation des compositions cosmétiques et pharmaceutiques de la présente invention, la température de fusion du composé cristallin encapsulé est comprise entre la température ambiante, c'est-à-dire environ 20 à 25 °C, et la température de la peau humaine c'est-à-dire environ 32 °C. A la température de conservation de ces compositions, le composé cristallin encapsulé dans les microcapsules est à l'état cristallin. Il fondra rapidement après application sur la peau ou les lèvres en puisant dans l'environnement cutané immédiat la chaleur nécessaire pour cette fusion et en créant ainsi chez l'utilisateur une sensation locale de fraîcheur.

Cet effet de fraîcheur, comparable à celui du menthol, n'implique cependant pas le contact direct entre le produit chimique et la peau et exclut ainsi tout risque d'irritation.

Dans un troisième de mode de réalisation des compositions cosmétiques et pharmaceutiques de la présente invention, la température de fusion du composé cristallin encapsulé est supérieure à la température de la peau humaine.

De telles compositions permettront - dans le domaine de température voisin du point de fusion du composé encapsulé - de prévenir les effets néfastes de la chaleur.

L'incorporation de microcapsules thermostabilisatrices renfermant un composé ayant un point de fusion légèrement supérieur à la température de la peau humaine, par exemple compris entre 33 et 45 °C, est particulièrement intéressante dans le domaine du maquillage. Lors d'une augmentation de la température extérieure, le composé cristallin encapsulé fondra en stabilisant localement et pendant un certain temps la température à une valeur proche de son point de fusion.

Cette stabilisation de la température améliore la tenue du maquillage et préserve ses qualités cosmétiques, par exemple la matité du teint, en l'empêchant de couler et en limitant localement la transpiration, voire le débit de sécrétion des glandes sébacées.

Il est bien entendu possible d'associer dans une même composition cosmétique plusieurs types de microcapsules qui diffèrent par le point de fusion du composé qu'elles renferment.

L'énumération ci-dessus des applications des compositions cosmétiques contenant les microcapsules thermostabilisatrices de la présente invention n'est bien entendu pas limitative. Les compositions cosmétiques "thermostatées" de la présente invention seront utiles dans toutes les applications où il s'agit d'apporter ou d'évacuer localement de la chaleur ou de stabiliser localement la température des surfaces (peau, lèvres, cheveux) sur lesquelles elles sont appliquées.

Les composés cristallins sont de préférence encapsulés dans une enveloppe étanche.

Cette encapsulation est une condition essentielle pour la réversibilité des processus de fusion/cristallisation. En effet, le composé fondu encapsulé ne pourra pas diffuser dans la composition cosmétique ou pharmaceutique et cristallisera de nouveau lorsque la température extérieure deviendra inférieure à son point de fusion.

L'étanchéité de l'enveloppe est intéressante en particulier lorsque la composition est susceptible d'être exposée à de nombreuses variations thermiques dans le domaine de fusion du composé cristallin.

Elle est indispensable par exemple dans des crèmes de protection contre le froid contenant, à température ambiante, le composé cristallin à l'état fondu.

Toutefois, dans certaines applications, notamment lorsqu'on recherche une stabilisation thermique ponctuelle par fusion du composé cristallin, la réversibilité des phénomènes de fusion/cristallisation n'est pas indispensable et l'imperméabilité de l'enveloppe vis-à-vis du composé cristallin fondu n'est pas une caractéristique essentielle de l'invention.

On peut citer à titre d'exemples de composés cristallins encapsulés appropriés pour la présente invention
- les hydrocarbures aliphatiques contenant de 10 à 40 atomes de carbone,
- les hydrocarbures aromatiques,
- les acides gras en C₈₋₄₀ tels que l'acide stéarique et l'acide laurique,
- les alcools gras en C₈₋₄₀ tels que l'alcool stéarylique et l'alcool laurylique,
- les esters des acides gras en C₁₀₋₄₀ tels que le stéarate de méthyle et le cinnamate de méthyle,
- les sels minéraux contenant une importante fraction d'eau de cristallisation, tels que l'hexahydrate de chlorure de calcium, le décahydrate de sulfate de sodium, le dodécahydrate d'hydrogénophosphate de sodium, le pentahydrate de thiosulfate de sodium et l'hexahydrate de nitrate de nickel,
- les triglycérides d'acides gras en C₁₀₋₄₀,
- certaines cires de silicone telles que les polydiméthylsiloxanes à groupements terminaux béhénoxy ou stéaroxy (INCI : behenoxydimethicone et stearoxydimethicone), les polyméthylstéaryloxy-diméthylsiloxanes (INCI : stearic ester dimethicone), les polyméthylstéaryl-diméthylsiloxanes (INCI : stéaryldimethicone), des copolymères à motifs méthacrylate de stéaryle avec greffons polydiméthylsiloxane, les polyméthyltrifluorométhylalkyl-diméthylsiloxanes (INCI : Trifluorométhyl(C₁₋₄)alkyldiméthicone)
les chaînes grasse de tous ces composés étant de préférence linéaires et/ou saturées.

Ces composés peuvent être utilisés seuls ou sous forme de mélange de deux ou plusieurs d'entre-eux.

On peut également citer les polymères thermofusibles au moins partiellement cristallins présentant un point de fusion cristalline dans la zone de température indiquée ci-dessus.

De tels polymères sont par exemple les homopolymères et copolymères oléfiniques, y compris les cires polyoléfiniques, tels que les homopolymères d'éthylène, les copolymères d'éthylène et de propylène, les copolymères d'éthylène et d'octène, les copolymères d'éthylène et de butène et les copolymères d'éthylène et d'acétate de vinyle.

On peut également citer les poly(alkylène oxyde), les polyesters d'alkyle, les poly(ε-caprolactones), les polyamides, en particulier ceux résultant de la polycondensation de dimère d'acides gras, ainsi que les copolymères d'oléfines fluorées.

Un autre groupe de polymères cristallins utilisables est formé par les polymères à chaînes latérales cristallisables décrits dans *J. Polymer Sci. : Macromol. Rev. 8:117-253 (1974).* Il s'agit de polymères ou copolymères vinyliques et/ou acryliques contenant une fraction importante, généralement au moins égale à 50 % en poids, de motifs copolymérisés comportant de longues chaînes latérales aliphatiques linéaires cristallisables, ou des chaînes latérales fluorées ou perfluorées cristallisables. Le brevet US 5 156 911 décrit l'utilisation de tels polymères à chaînes latérales cristallisables dans des assemblages adhésifs dont les propriétés adhésives varient en fonction de la température.

Les composés cristallins encapsulés préférés pour la présente invention sont les hydrocarbures aliphatiques à chaîne linéaire contenant de 10 à 40, de préférence de 13 à 28 et mieux encore de 16 à 23 atomes de carbone. En effet, la température de fusion des composés de cette série d'hydrocarbures homologues augmente de manière parfaitement prévisible avec le nombre d'atomes de carbone (-5,5 °C pour le n-tridécane (C₁₃) à 61,4 °C pour le n-octacosane (C₂₈)) dans le domaine de température concerné par les applications cosmétiques décrites ci-dessus.

Les composés cristallins présentant une enthalpie de fusion (ΔH_{f}) comprise entre 75 et 330 kJ/kg sont de préférence encapsulés dans une enveloppe étanche.

Cette encapsulation est une condition essentielle pour la réversibilité des processus de fusion/cristallisation. En effet, le composé fondu encapsulé ne pourra pas diffuser dans la composition cosmétique et cristallisera de nouveau lorsque la température extérieure deviendra inférieure à son point de fusion.

Le matériau formant la paroi des microcapsules peut être choisi parmi tous les matériaux classiquement utilisés dans le domaine de la microencapsulation.

Ce matériau peut être amorphe, cristallin ou semi-cristallin. Lorsqu'il est cristallin ou semi-cristallin, il doit avoir un point de fusion supérieur à celui des composés cristallins encapsulés. Par ailleurs, ce matériau doit être suffisamment élastique pour résister aux variations de volume du composé cristallin lors de la transition de phase et pour résister aux forces de cisaillement lors de l'application de la composition le contenant. De plus, il doit être inerte vis-à-vis des substances encapsulées et des composés de la formulation cosmétique ou pharmaceutique avec laquelle il sera en contact.

Selon le procédé choisi, on pourra utiliser comme matériaux des polymères tels que les polyamides, les polyuréthanes, les polyurées, les polyesters, les polycyanoacrylates, les résines urée-formaldéhyde ou mélamine-formaldéhyde, et les systèmes gélatine/gomme arabique.

Les microcapsules peuvent être préparées selon des procédés bien connus décrits par exemple dans l'ouvrage intitulé "*Microencapsulation, Methods and Industrial Applications",* Directeur de la publication S. Benita, Marcel Dekker (1996). On peut citer à titre d'exemple la polymérisation ou polycondensation interfaciale, la coacervation, l'atomisation, l'extrusion centrifuge ou la microencapsulation sur disques rotatifs.

Les microcapsules thermostabilisatrices sont connues et commercialisées par exemple sous la dénomination Thermasorb® 'par la société Frisby Technologies Inc. ou sous les références 9850K et 9850Q par la société 3M.

Ces microcapsules se présentent sous forme d'une poudre fine, fluide et non filmogène. Leur utilisation est connue par exemple dans le domaine des vêtements et chaussures isothermes, dans des systèmes de refroidissement en microélectronique et dans le domaine de l'emballage.

On peut également utiliser comme composé constituant la paroi des microparticules un matériau non polymérique. On peut utiliser par exemple des microcapsules à base de silice précipitée, amorphe, hydratée ou rendue hydrophobe, proposées par la société PHASE CHANGE LABORATORIES sous la dénomination AcuTemp® .

La taille supérieure des microcapsules est de préférence limitée, pour des raisons évidentes de visibilité, à quelques dizaines ou centaines de micromètres. On préfère généralement utiliser des microcapsules ayant un diamètre moyen compris entre 0,01 et 100 micromètres, mieux encore entre 0,05 et 50 micromètres.

La proportion des microcapsules dans les compositions cosmétiques ou pharmaceutiques de la présente invention peut varier entre de larges limites qui dépendent de la formulation et de l'application envisagée.

Les compositions cosmétiques de la présente invention contiennent généralement de 1 % à 99 % en poids, et de préférence de 5 % à 90 % en poids de microcapsules thermostabilisatrices, rapporté à la composition cosmétique ou pharmaceutique finale.

Les compositions cosmétiques ou pharmaceutiques peuvent contenir en outre des principes actifs cosmétiques et/ou pharmaceutiques et des adjuvants appropriés pour l'application envisagée. Elles contiennent de préférence au moins un matériau à propriétés optiques. Celui-ci peut être choisi par exemple parmi les colorants hydrosolubles ou liposolubles, les pigments blancs ou colorés, les laques, les poudres de polymère, les nacres ou les paillettes.

Ces matériaux sont de préférence présents dans les compositions de la présente invention en une quantité comprise entre 0,1 et 99 % en poids de préférence entre 0,5 et 90 % en poids.

On peut citer à titre d'exemples de principes actifs cosmétiques les agents anti-radicaux libres, les agents hydratants, les vitamines, les protéines, les enzymes, les céramides, les α-hydroxyacides, les β-hydroxyacides, les rétinoïdes, les filtres solaires, les agents tensioactifs, les agents de matité, les agents anti-transpiration, les agents bactéricides et bactériostatiques, les corps gras et les silicones.

Les adjuvants sont par exemple les solvants, les agents régulateurs de pH, les anti-oxydants, les agents séquestrants, les agents conservateurs, les charges, les émollients, les agents anti-mousse, les corps gras tels que les huiles, les cires et les corps gras pâteux, les agents dispersants, les silicones telles que les huiles volatiles ou non, les gommes, les cires ou les silicones pâteuses, les parfums, les agents tensioactifs, les plastifiants, les polymères épaississants ou gélifiants, et les polymères filmogènes, solubles ou dispersibles.

Les compositions cosmétiques ou pharmaceutiques de la présente invention peuvent se présenter sous n'importe quelle forme connue compatible avec la présence des microcapsules thermostabilisatrices. Elles peuvent être par exemple sous forme de suspension aqueuse, hydro-alcoolique ou organique, sous forme d'émulsion huile-dans-eau ou eau-dans-huile ou encore sous forme d'émulsion multiple, sous forme de gel aqueux ou huileux, de pâte, de poudre libre ou compacte, ou de stick.

La présente invention a également pour objet des substrats solides insolubles, imprégnés avec une composition cosmétique ou pharmaceutique telle que décrite ci-dessus.

En effet, les compositions cosmétiques ou pharmaceutiques de la présente invention peuvent également servir à imprégner un substrat solide insoluble. Le substrat insoluble peut être choisi dans le groupe comprenant les matériaux textiles tissés ou non tissés, les mousses, les éponges, les ouates ou les billes. Il peut s'agir notamment d'un substrat textile non-tissé à base de fibres d'origine naturelle telles que les fibres de lin, de coton ou de soie, ou d'origine synthétique telles que les fibres de cellulose, de viscose, de polymères vinyliques, de polyesters comme le poly(téréphtalate d'éthylène), de polyoléfines comme le polyéthylène ou le polypropylène, de polyamides comme le Nylon® ou de polymères acryliques. Les matériaux non-tissés sont décrits par exemple dans "Nonwoven Binding Methods & Materials", de Riedel, Nonwoven World, 1987. Ces substrats sont obtenus selon des procédés connus dans le domaine de la technique de préparation des non-tissés.

Ce substrat peut comporter une ou plusieurs couches ayant des propriétés identiques ou différentes, et qui peuvent apporter par exemple des propriétés d'élasticité ou de douceur selon l'usage envisagé. Les substrats peuvent par exemple comporter deux parties ayant des propriétés d'élasticité différentes, comme ceux décrits dans la demande internationale WO99/13861 ou bien comporter une seule couche avec des densités différentes comme décrit dans le document WO 99/25318, ou encore comporter deux couches de texture différente comme les substrats décrits par exemple dans la demande internationale WO 98/18441.

Le substrat peut avoir n'importe quelle taille ou forme appropriée à l'application envisagée.

Il a généralement une surface comprise entre 0,005 m² et 0,1 m², de préférence entre 0,01 m² et 0,05 m². Il se présente de préférence sous la forme de lingettes rectangulaires ou de compresses rondes.

L'article final comportant le substrat et la composition d'imprégnation est généralement à l'état humide, avec un taux d'imprégnation, c'est-à-dire une quantité de composition rapportée au poids du substrat solide, compris entre 200 et 1000 %, de préférence entre 250 et 350 %.

Les techniques d'imprégnation des substrats sont bien connues dans le domaine technique et sont toutes applicables à la présente invention. Le plus souvent, la composition d'imprégnation est ajoutée au substrat par une ou plusieurs techniques telles que l'immersion, l'enduction ou la vaporisation.

Il est également possible de constituer un article (ou lingette) présenté à l'état sec soit en éliminant l'eau de la composition après son imprégnation sur le substrat, soit en déposant sur le substrat une composition sous forme sèche à l'état de poudre, granule ou film, par tous les moyens de réalisation connus tels que le soudage et le collage de multicouches par voie thermique ou par ultrasons. Dans ce dernier mode de réalisation, la composition est séchée par tout moyen connu, par exemple par atomisation, lyophilisation ou autre technique analogue.

On peut ainsi obtenir, selon l'utilisation envisagées, des lingettes humides ou des lingettes sèches. Les lingettes humides peuvent être utilisées telles quelles alors que les lingettes sèches sont mouillées avant utilisation.

Comme indiqué ci-dessus les applications potentielles des compositions contenant des microcapsules thermostabilisatrices de la présente invention sont nombreuses.

Il peut s'agir par exemple de compositions de maquillage telles que les fonds de teint, poudres, mascaras, eye-liners, rouges à lèvres ou produits de maquillage des cheveux, de produits déodorants notamment sous forme de sticks ou de crème, de crèmes de protection, de crèmes de soin ou de produits de soins capillaires.

Les articles ou lingettes à support solide insoluble décrits ci-dessus peuvent être notamment des articles ou lingettes approprié(e)s pour le soin etou le traitement de la peau et notamment une lingette de nettoyage ou de démaquillage de la peau du visage et/ou du corps, et/ou une lingette de nettoyage ou de démaquillage des yeux, ainsi que pour le nettoyage des peaux à tendance grasse ou acnéique.

Comme indiquée ci-dessus, l'effet tampon thermique des microcapsules thermostabilisatrices utilisées dans la présente invention est particulièrement intéressant dans le cas de compositions susceptibles de subir une altération réversible ou irréversible de leur propriétés suite à un changement de température. On peut citer à titre d'exemple de telles compositions les sticks déodorants, les compositions parfumantes solides, les rouges à lèvres, les crayons de maquillage, les crèmes solaires, les mascaras, les crèmes de soin, les gels aqueux ou huileux, et les lingettes imprégnées d'une émulsion huile dans eau telles que les lingettes démaquillantes.

Les exemples ci-dessous illustrent la présente invention sans pour autant la limiter.

### Exemple 1

### Stabilisation thermique d'une composition cosmétique

On observe l'évolution de la stabilité de sticks anhydres en fonction de la température extérieure. Les sticks testés ont la composition suivante :

| Stick A (témoin) : | |
|---|---|
| Lanoline | 24 % en poids |
| Dérivé de lanoline | 5,95 % en poids |
| Cire de lanoline | 6 % en poids |
| Ester d'acide gras | 4 % en poids |
| Argile modifiée | 0,6 % en poids |
| Pigments | 8,66 % en poids |
| Agent antioxydant | 0,6 % en poids |
| Parfum | 0,2 % en poids |
| Huile végétale | qsp. 100 % en poids |

### Stick B (selon l'invention) :

+ 35 % en poids de microcapsules contenant de l'eicosane.
65 % en poids de la composition formant le stick A

On conserve les sticks dans une étuve thermostatée à 47 °C, on les retire de l'étuve à des intervalles de 10 minutes et on mesure immédiatement la température de chacun des sticks à l'aide d'un thermomètre IR (modèle Infratherm de la société Chauvin/Arnoux, France).
On obtient les résultats suivant :

| | T° du stick à T₀ | T° du stick après 10 min | T° du stick après 20 min | T° du stick après 30 min |
|---|---|---|---|---|
| Stick A (témoin) | 27,6 °C | 35,0 °C | 37,7 °C | 38,5 °C |
| Stick B (invention) | 27,4 °C | 32,3 °C | 34,2 °C | 36,4 °C |

Ces résultats montrent que la présence des microcapsules permet de ralentir la montée en température du stick.

### Exemple 2

On prépare une poudre libre en mélangeant les ingrédients suivants :

| | |
|---|---|
| Microcapsules 9850Q¹⁾ | 40 parties en poids |
| Talc | 44 parties en poids |
| Poudre de nylon | 10 parties en poids |
| Oxyde de fer jaune | 1,6 partie en poids |
| Oxyde de fer rouge | 1,0 partie en poids |
| Oxyde de fer noir | 0,4 partie en poids |
| Diméthicone²⁾ | 3 parties en poids |

| | |
|---|---|
| ¹⁾microcapsules commercialisées par la société 3M, renfermant un composé avec un point de fusion à 34 °C. | |
| ²⁾ huile de polydiméthylsiloxane non volatile | |

La poudre libre ainsi préparée permet de maintenir une bonne matité du teint à des températures extérieures relativement élevées.

### Exemple 3

On prépare une poudre libre en mélangeant les ingrédients suivants :

| | |
|---|---|
| Microcapsules THERMASORB® 83¹⁾ | 40 parties en poids |
| Talc | 44 parties en poids |
| Poudre de nylon | 10 parties en poids |
| Oxyde de fer jaune | 1,6 partie en poids |
| Oxyde de fer rouge | 1,0 partie en poids |
| Oxyde de fer noir | 0,4 partie en poids |
| Diméthicone²⁾ | 3 parties en poids |

| | |
|---|---|
| ¹⁾ microcapsules commercialisées par la société FRISBY TECHNOLOGIES, renfermant un composé avec un point de fusion à 28 °C. | |
| ²⁾ huile de polydiméthylsiloxane non volatile | |

Lors de l'application de la poudre on constate un effet de fraîcheur immédiat.

### Exemple 4

### Composition de lait démaquillant pour lingettes

| Phase A | |
|---|---|
| Stéarate de glycéryle/stéarate de PEG 100 (Arlacel® 165) | 0,55 % |
| Alcool cétylique | 0,15 % |
| Gomme de xanthane | 0,1 % |
| Palmitate d'isopropyle | 3,8 % |

| Phase B | |
|---|---|
| Eau | q.s.p. 100 % |
| Conservateurs (parabens phenoxyethanol) | 0,65 % |

| Phase C | |
|---|---|
| Microcapsules thermostabilisatrices | 10 % |

On chauffe séparément les phases A et B à une température comprise entre 75 et 80 °C. On ajoute la phase A dans laphase B sous agitation, on maintient l'agitation pendant 5 minutes, puis on ajoute sous faible agitation la phase C. On laisse refroidir à température ambiante tout en maintenant une faible agitation. On obtient ainsi un lait à faible teneur en polymère épaississant présentant une bonne résistance au crêmage à température élevée. Ce lait peut être utilisé pour l'imprégnation de lingettes démaquillantes selon des techniques usuelles d'imprégnation.

## Revendications

1. Composition cosmétique ou pharmaceutique comprenant, dans un support physiologiquement acceptable, des microcapsules renfermant au moins un composé cristallin présentant une enthalpie de fusion (ΔH_{f}), mesurée par analyse enthalpique différentielle, comprise entre 75 et 330 kJ/kg.

2. Composition cosmétique ou pharmaceutique selon la revendication 1, **caractérisée par le fait que** les microcapsules ont une enveloppe étanche, imperméable au composé cristallin à l'état fondu.

3. Composition cosmétique ou pharmaceutique selon la revendication 1 ou 2, **caractérisée par le fait que** l'enthalpie de fusion du composé cristallin est comprise entre 100 et 300 kJ/kg, de préférence entre 150 et 280 kJ/kg.

4. Composition cosmétique ou pharmaceutique selon l'une quelconque des revendications précedentes, **caractérisée par le fait que** la température de fusion (T_{f}) du composé cristallin encapsulé est comprise entre 0 °C et 90 °C.

5. Composition cosmétique ou pharmaceutique selon la revendication 4, **caractérisée par le fait que** la température de fusion du composé cristallin encapsulé est comprise entre 5 et 15 °C.

6. Composition cosmétique ou pharmaceutique selon la revendication 4, **caractérisée par le fait que** la température de fusion du composé cristallin encapsulé est comprise entre 25 et 60 °C.

7. Composition cosmétique ou pharmaceutiques selon la revendication 4, **caractérisée par le fait que** la température de fusion (T_{f}) du composé cristallin encapsulé est inférieure à la température ambiante, de préférence comprise entre 0 et 15 °C.

8. Composition cosmétique ou pharmaceutique selon la revendication 4, **caractérisée par le fait que** la température de fusion du composé cristallin encapsulé est comprise entre la température ambiante et la température de la peau humaine (32 °C).

9. Composition cosmétique ou pharmaceutique selon la revendication 4, **caractérisée par le fait que** la température de fusion du composé cristallin encapsulé est supérieure à la température de la peau humaine.

10. Composition cosmétique ou pharmaceutique selon l'une quelconque des revendications précédentes, **caractérisée par le fait que** le composé cristallin encapsulé est choisi parmi
- les hydrocarbures aliphatiques contenant de 10 à 40 atomes de carbone,
- les hydrocarbures aromatiques,
- les acides gras en C₈₋₄₀,
- les alcools gras en C₈₋₄₀,
- les esters des acides gras en C₁₀₋₄₀,
- les sels minéraux contenant une importante fraction d'eau de cristallisation;
- les triglycérides d'acides gras en C₁₀₋₄₀, et
- les cires de silicone,
les chaînes grasses de ces composés étant de préférence linéaires et/ou saturées, et
- les polymères thermofusibles cristallins.

11. Composition cosmétique ou pharmaceutique selon l'une quelconque des revendications précédentes, **caractérisée par le fait que** le composé cristallin est un hydrocarbure aliphatique à chaîne linéaire et saturée contenant de 10 à 40 atomes de carbone, de préférence de 13 à 28 atomes de carbone, et mieux encore de 16 à 23 atomes de carbone.

12. Composition cosmétique ou pharmaceutique selon l'une quelconque des revendications précédentes, **caractérisée par le fait que** le matériau formant l'enveloppe est choisi parmi les polyamides, les polyuréthannes, les polyurées, les polyesters, les polycyanoacrylates, les résines urée-formaldéhyde ou mélamine-formaldéhyde, les systèmes gélatine/gomme arabique et la silice.

13. Composition cosmétique ou pharmaceutique selon l'une quelconque des revendications précédentes, **caractérisée par le fait que** les microcapsules ont un diamètre moyen compris entre 0,01 et 100 micromètres, de préférence entre 0,05 et 50 micromètres.

14. Composition cosmétique ou pharmaceutique selon l'une quelconque des revendication précédentes, **caractérisée par le fait que** les microcapsules représentent de 1 à 99 % en poids, de préférence de 5 à 90 % en poids de la composition finale.

15. Composition cosmétique ou pharmaceutique selon la revendication 1, **caractérisé par le fait qu'**elle contient en outre au moins un matériau à propriétés optiques.

16. Composition cosmétique ou pharmaceutique selon la revendication 15, **caractérisée par le fait que** le matériau à propriétés optiques est présent à raison de 0,1 à 99 % en poids, rapporté à la composition cosmétique finale.

17. Composition cosmétique ou pharmaceutique selon l'une quelconque des revendications précédentes, **caractérisée par le fait qu'**il s'agit d'une composition de maquillage, d'un produit déodorant, d'une crème de protection, d'une crème de soin ou d'un produit de soin capillaire.

18. Utilisation de microcapsules renfermant au moins un composé cristallin présentant une enthalpie de fusion (ΔH_{f}), mesurée par analyse enthalpique différentielle, comprise entre 75 et 330 kJ/kg, pour protéger des compositions cosmétiques ou pharmaceutiques contre les effets de variations thermiques.

19. Utilisation selon la revendication 18, **caractérisée par le fait que** les microcapsules ont une enveloppe étanche, imperméable au composé cristallin à l'état fondu.

20. Utilisation selon la revendication 18 ou 19, **caractérisée par le fait que** l'enthalpie de fusion du composé cristallin est comprise entre 100 et 300 kJ/kg, de préférence entre 150 et 280 kJ/kg.

21. Utilisation selon une des revendications 18 à 20, **caractérisée par le fait que** la température de fusion (T_{f}) du composé cristallin encapsulé est comprise entre 0 °C et 90 °C.

22. Utilisation selon la revendication 21, **caractérisée par le fait que** la température de fusion du composé cristallin encapsulé est comprise entre 5 et 15 °C.

23. Utilisation selon la revendication 21, **caractérisée par le fait que** la température de fusion du composé cristallin encapsulé est comprise entre 25 et 60 °C.

24. Utilisation selon l'une quelconque des revendications 18 à 23, **caractérisée par le fait que** le composé cristallin encapsulé est choisi parmi
- les hydrocarbures aliphatiques contenant de 10 à 40 atomes de carbone,
- les hydrocarbures aromatiques,
- les acides gras en C₈₋₄₀,
- les alcools gras en C₈₋₄₀,
- les esters des acides gras en C₁₀₋₄₀,
- les sels minéraux contenant une importante fraction d'eau de cristallisation,
- les triglycérides d'acides gras en C₁₀₋₄₀, et
- les cires de silicone,
les chaînes grasses de ces composés étant de préférence linéaires et/ou saturées, et
- les polymères thermofusibles cristallins.

25. Utilisation selon l'une des revendications 18 à 24, **caractérisée par le fait que** le composé cristallin est un hydrocarbure aliphatique à chaîne linéaire contenant de 10 à 40 atomes de carbone, de préférence de 13 à 28 atomes de carbone et mieux encore de 16 à 23 atomes de carbone.

26. Utilisation selon l'une des revendications 18 à 25, **caractérisé par le fait que** le matériau formant l'enveloppe est choisi parmi les polyamides, les polyuréthannes, les polyurées, les polyesters, les polycyanoacrylates, les résines urée-formaldéhyde ou mélamine-formaldéhyde, les systèmes gélatine/gomme arabique et la silice.

27. Utilisation selon l'une des revendications 18 à 26, **caractérisée par le fait que** les microcapsules ont un diamètre moyen compris entre 0,01 et 100 micromètres, de préférence entre 0,05 et 50 micromètres.

28. Substrat solide insoluble imprégné avec une composition cosmétique ou pharmaceutique selon l'une quelconque des revendications 1 à 17.

29. Substrat solide insoluble selon la revendication 28, **caractérisé par le fait qu'**il s'agit d'un substrat textile non tissé à base de fibres d'origine naturelle ou synthétique.

30. Substrat solide insoluble selon l'une des revendications 28 ou 29, **caractérisé par le fait qu'**il a une surface comprise entre 0,005 m² et 0,1 m², de préférence entre 0,01 m² et 0,05 m².

31. Substrat solide insoluble selon l'une des revendications 28 à 30, **caractérisé par le fait que** le taux d'imprégnation, c'est-à-dire la quantité de composition cosmétique ou pharmaceutique rapportée au poids du substrat solide, est compris entre 200 % et 1000 %, de préférence entre 250 % et 350 %.

32. Substrat solide insoluble selon l'une des revendications 28 à 31, **caractérisé par le fait qu'**il se présente sous la forme de lingettes rectangulaires ou de compresses rondes.
